# EUROPÄISCHE PATENTSCHRIFT

(11) **EP 2 392 284 B1**
(45) Veröffentlichungstag und Bekanntmachung des Hinweises auf die Patenterteilung: **19.12.2018**
(21) Anmeldenummer: 11004528.3
(22) Anmeldetag: 01.06.2011
(51) Int. Cl.: A61B 90/00, A61B 50/20, A61B 50/30, A61B 17/86

(54) **Kennzeichnungssystem für medizinische Implantate**
Identification system for medical implants
Système d'identification pour implants médicaux

(30) Priorität: 01.06.2010 DE 202010007487 U
(43) Veröffentlichungstag der Anmeldung: 07.12.2011
(73) Patentinhaber: Innovations Medical GmbH, 78532 Tuttlingen (DE)
(72) Erfinder: Kreidler, Winfried, 78532 Tuttlingen (DE)

(56) Entgegenhaltungen:
- WO-A1-2005/092231
- WO-A2-2009/024189
- DE-B3-102007 011 093
- DE-U1-202007 004 638
- US-A1- 2002 052 607

## Beschreibung

Die Erfindung betrifft ein Kennzeichnungssystem für medizinische Implantate, bei dem auf einer Beschriftungsfläche produktspezifische Daten, wie z. B. Hersteller, Charge oder sonstige Kennzeichnungsdaten unlöschbar aufgebracht sind, anhand derer die Herkunft oder die Eigenschaften oder die Herkunft und die Eigenschaften des betreffenden Implantats rückverfolgbar sind, wobei sich die Beschriftungsfläche auf einem Datenträger befindet, der die Form eines Plättchens, Schildchens oder Streifens aufweist.

Bei den bekannten Kennzeichnungssystemen sind die Kennzeichnungsdaten eines Implantats unmittelbar auf dem Implantat selbst und/oder auf der Verpackung angebracht. Aus der DE 20 2007 004 638 U1 ist beispielsweise eine Haltevorrichtung für die Aufnahme von Knochenschrauben bekannt, bei welcher auf einer Sichtfläche Implantatdaten angeordnet sind. Diese Haltevorrichtung weist einen Grundkörper auf, in welchen das Implantat über eine Art Rast- oder Klemmverbindung auslösbar eingesetzt ist. Mehrere solcher Haltevorrichtungen können zusammen mit dem aufgenommenen Implantat in eine Lagereinheit lösbar eingesetzt sein, wozu die Haltevorrichtung jeweils mit weiteren Rastelementen versehen ist. Für den Einsatz eines Implantates beispielsweise im menschlichen Körper wird zunächst die Haltevorrichtung aus der Lagereinheit entnommen. Anschließend wird das Implantat aus der Haltevorrichtung herausgenommen und in den Körper des Patienten eingesetzt. Weil das Implantat nach dem Einsetzen in den Körper des Patienten nicht mehr zugänglich ist und die Haltevorrichtung aufgrund ihrer Abmessungen nicht archivierbar ist, müssen die Daten zur Archivierung und Zuordnung zum jeweiligen Patienten von der Haltevorrichtung abgeschrieben und in die Patientenakte übertragen werden. Dabei können Übertragungsfehler entstehen, so dass eine zuverlässige Rückverfolgung nicht mit der erforderlichen Sicherheit gewährleistet ist.

In der US 2002/0052607 A1 ist ein Implantat in Form einer Schädelplatte beschrieben an welcher eine Art Schildchen direkt angeformt ist. Dieses Schildchen bildet auf seiner dem Schädel des Patienten abgewandten Außenseite eine Beschriftungsfläche, welche diverse Daten der Schädelplatte, wie Nummer, Größe, Material und andere zugehörige Informationen enthält. Des Weiteren dient dieses Schildchen zur Erleichterung der manuellen Positionierung. Das Schildchen steht über eine "zerbrechliche" Verbindungsstelle mit der Schädelplatte in Verbindung, so dass das Schildchen, nachdem das Implantat am Schädel positioniert und befestigt wurde, manuell entfernt werden kann.

Beim Gegenstand der DE 10 2007 011 093 B3 geht es um einen separaten Datenträger, welcher mit einem Implantat in Form einer Knochenplatte ein Eingriff bringbar ist. Hierzu ist der Datenträger mit einem blattfederartigen Betätigungselement versehen, das einen mittleren, aus der Ebene des Datenträgers hervorstehenden Betätigungsabschnitt aufweist. Der freie federelastisch bewegliche Endbereich des Betätigungselement ist "unterhalb" der Ebene des Datenträgers angeordnet und weist oberseitig einen Haltezapfen auf, welcher in eine der Bohrungen der Knochplatte einsetzbar ist. Zu beiden Seiten des Betätigungselementes sind U-Schenkel vorgesehen, welche sich seitlich bis in den Bereich des Haltezapfens erstrecken und mit ihren Endbereichen als an der Knochenplatte oberseitig ansetzbare Widerlager im Bereich des Haltezapfens dienen. Durch Betätigen der "Betätigungsabschnittes" kann der Haltezapfen mit der Bohrungen des Implantates außer Eingriff gebracht und vom Implantat entfernt werden. werden. Auf Grund dieser Abmessungen ist dieser Datenträger ebenfalls nicht archivierbar, insbesondere nicht in einer Patentenakte.

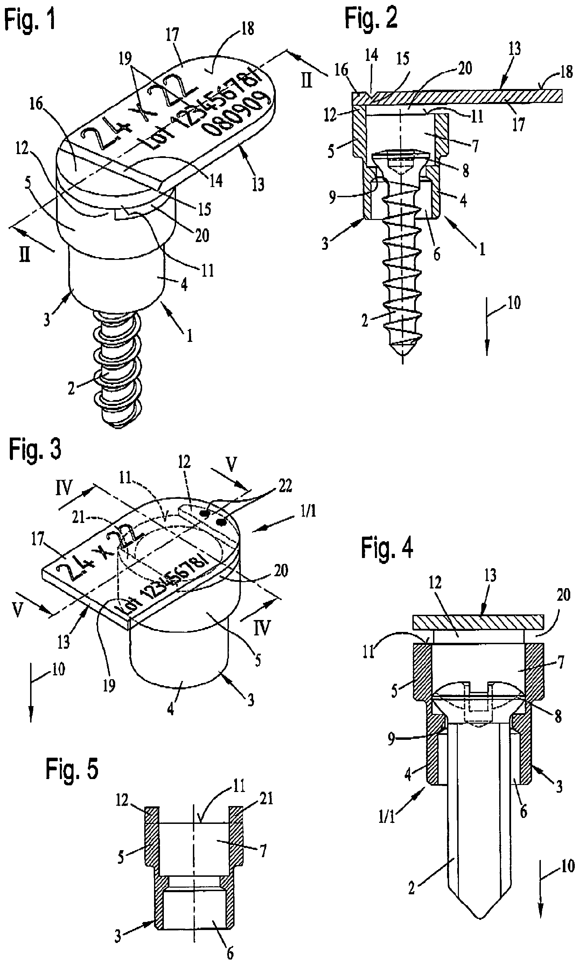

Beim Gegenstand der WO 2009/024189 A2 geht es um die Kennzeichnung von Implantaten, insbesondere in Form einer Implantatschraube. Bei dieser Vorrichtung ist Lagerelement vorgesehen, an welchem zwei die Datenträger angeformt sind. Einer der Datenträger verläuft unterseitig parallel zur Achse einer aufzunehmenden Knochenschraube, während der zweite Datenträge als oberseitig seitlich abstehendes Element ausgebildet ist. Diesem Lagerelement 5 ist ein Aufnahmeelement 3 zugeordnet, welches in das Lagerelement einsteckbar ist und welches die Knochenschraube aufnimmt. Befindet sich dieses Aufnahmeelement im Lagerelement in einer Ausgangsstellung, so wird die Knochenschraube durch einen Haltebügel rastend sowohl im Aufnahmeelement als auch im Lagerelement gesichert. Durch Niederdrücken des Aufnahmeelementes relativ zum Lagerelement wird der Haltebügel in die in den Fig. 2a bis 2e dargestellte Position zurückgestellt. In dieser Position, in welcher das Aufnahmeelement in das Lagerelement hinein verschoben wurde, wird die Knochenschraube durch den Haltebügel frei gegeben, so dass diese aus dem Aufnahmeelement herausgenommen werden kann. Von dem abgesehen, dass diese Vorrichtung zum Kennzeichnen einer Knochenschraube äußerst komplex aufgebaut ist, so ist auch diese Vorrichtung nicht in einer Patientenakte archivierbar.

Der Erfindung liegt demgemäß die Aufgabe zugrunde, ein Kennzeichnungssystem der eingangs genannten Art zu schaffen, in welchem ein Implantat einfach und sicher aufnehmbar ist und bei dem die Daten des benutzten Implantats in der vom Hersteller des Implantats angegebenen Form und Vollständigkeit in die Patientenakte aufnehmbar sind, um Übertragungsfehler zu vermeiden.

Die Aufgabe wird erfindungsgemäß zusammen mit den Merkmalen des Oberbegriffes des Anspruches 1 dadurch gelöst, dass der Datenträger manuell abtrennbar mit einem das Implantat aufnehmenden Lagerelement verbunden ist und das Implantat im Lagerelement durch den Datenträger unverlierbar gesichert ist und, dass der abgetrennte Datenträger an oder auf einem Patientendokument mittels einer Kleb- oder einer Klammerverbindung unverlierbar befestigbar ist.

Ein Datenträger in Form eines dünnen Plättchens, Schildchens oder Streifens lässt sich beispielsweise durch Kleben oder mittels Heftklammern leicht an einem aus Papier oder leichtem Karton bestehenden Patientendokument befestigen und zusammen mit diesem zur Archivierung aufbewahren.

Mit einem solchen Kennzeichnungssystem, das leicht und ohne besondere Aufmerksamkeit sicher handhabbar ist, können Datenübertragungsfehler mit der gebotenen Sicherheit vermieden und die Daten der Implantate unverlierbar über längere Zeiträume zurückverfolgt werden.

Ein besonderer Vorteil besteht auch darin, dass sich das erfindungsgemäße Kennzeichnungssystem sowohl für plattenförmige Implantate als auch für Implantate in Form von Implantatschrauben in gleich vorteilhafter Weise verwenden lässt.

Hierzu kann zur Magazinierung von mehreren Aufnahmehülsen oder Implantatschrauben ein Aufnahmemagazin oder ein Datenträgermagazin vorgesehen sein, das zur Aufnahme der Aufnahmehülsen oder der mit einem Datenträger versehenen Implantatschrauben mehrere Aufnahmebohrungen oder Durchgangsbohrungen aufweist.

Durch die erfindungsgemäßen Ausgestaltungen der Datenträger wird sichergestellt, dass ein Datenträger erst dann von der Implantatschraube bzw. deren Aufnahmehülse entfernt wird, wenn die Implantatschraube aus ihrer Aufnahmehülse entnommen wird. Damit ist eine erhöhte Kennzeichnungssicherheit gewährleistet.

Weiter kann vorgesehen sein, dass der Verbindungsabschnitt der Aufnahmehülse sich an einer Stirnseite der Aufnahmehülse befindet und dass der Verbindungsabschnitt eine durch eine kerbenartige Vertiefung erzeugte Querschnittsverjüngung aufweist. Durch diese Ausgestaltung ist der Datenträger an der einen Stirnseite der Aufnahmehülse leicht zugänglich und kann durch die kerbenartige Vertiefung des Verbindungsabschnittes auch einfach manuell abgetrennt werden. Durch die Anordnung des Verbindungsabschnitts und damit auch des Datenträgers wird auch die Übersichtlichkeit erleichtert und damit die Handhabung verbessert.

Zur einfachen Magazinierung von Implantatschrauben kann weiter vorgesehen sein, dass das Datenträgermagazin aus einem wenigstens eine Reihe von Durchgangsbohrungen für Implantatschrauben aufweisenden länglichen Block besteht, bei dem die Durchgangsbohrungen oberseitig durch einzelne, separat abtrennbare, den in den Bohrungen befindlichen Implantatschrauben zugeordneten Datenträger abgedeckt sind, deren Verbindungsabschnitte oberseitig außerhalb der Bohrungen liegen.

Weiter kann der Datenträger und das Teil, mit dem er abreißbar verbunden ist aus Kunststoff bestehen. Ergänzend hierzu kann der Verbindungsabschnitt als Filmscharnier ausgebildet sein. Durch diese Ausgestaltungen kann ein Handhabungsvorteil insofern erzielt werden, als die Möglichkeit geschaffen wird, durch Hochklappen des Datenträgers die Einsteckbohrungen zum Einlegen einer Implantatschraube zu öffnen.

Weiter kann vorgesehen sein, dass jeder Bohrung des aus Kunststoff bestehenden Datenträgermagazins zu deren Abdeckung ein als Deckel ausgebildeter Datenträger zugeordnet ist, der mittels eines Filmscharniers an einer Längskante eines das Datenträgermagazin bildenden Blocks aufklappbar und abreißbar angelenkt ist.

Diese Ausgestaltung ist für die Magazinierung von mehreren Implantatschrauben in einem gemeinsamen Datenträgermagazin äußerst vorteilhaft. Insbesondere durch Anlenkung des als Deckel ausgebildeten Datenträgers über ein Filmscharnier am Datenträgermagazin ist ein Öffnen des Deckels (bzw. der Deckel) zur Bestückung des Datenträgermagazins in ebenso einfacher Weise durchführbar wie auch das abschließende Entfernen des Datenträgers.

Zur Sicherung der Implantatschrauben im Datenträgermagazin kann vorgesehen sein, dass die Datenträger des Datenträgermagazins mittels eines abreißbaren Rastlasche in ihrer Schließlage am Datenträgermagazin verriegelbar sind.

Darüber hinaus kann auch vorgesehen sein, dass die als schwenkbarer Deckel ausgebildeten Datenträger des Datenträgermagazins jeweils mittels einer an der Stirnfläche des Datenträgermagazins angeordneten Rastlasche in ihrer Schließlage fixierbar sind, wobei die Rastlasche den jeweils zugeordneten Datenträger mit einer Rastnase übergreift.

Um eine einzelne Implantatschraube im Operationsbereich bereitzustellen, kann vorgesehen sein, dass die Kennzeichnungshülse einen axial verlängerten Standfuß mit einer unterseitigen, im Wesentlichen ebenen, etwa ringförmig ausgebildeten Standfläche aufweist.

Anhand der Zeichnungen werden nachfolgend einige Ausführungsbeispiele der Erfindung näher erläutert. Es zeigt:
- Fig. 1: eine perspektivische Darstellung einer ersten Ausführungsvariante eines Kennzeichnungssystems zusammen mit einem als Implantatschraube ausgebildeten Implantat;
- Fig. 2: eine Schnittdarstellung II-II des Kennzeichnungssystems aus Fig. 1;
- Fig. 3: eine zweite Ausführungsvariante eines Kennzeichnungssystems ohne Implantat;
- Fig. 4: eine Schnittdarstellung IV-IV des Kennzeichnungssystems aus Fig. 3 mit eingesetztem, als Implantatschraube ausgebildeten Implantat;
- Fig. 5: eine Schnittdarstellung V-V des Kennzeichnungssystems aus Fig. 3 mit entferntem Datenträger;
- Fig. 6: ein plattenförmiges Aufnahmemagazin mit mehreren Aufnahmebohrungen zusammen mit einem Kennzeichnungssystem aus Fig. 1, welches in eine der Aufnahmebohrungen eingesetzt ist;
- Fig. 7: eine Schnittdarstellung VII-VII aus Fig. 6 mit mehreren in die Aufnahmebohrungen des Aufnahmemagazins eingesetzten Kennzeichnungssystemen aus Fig. 1;
- Fig. 8: eine perspektivische Darstellung einer weiteren Ausführungsvariante eines Kennzeichnungssystems, welches mit einem Standfuß versehen ist;
- Fig. 9: eine Schnittdarstellung IX-IX des Kennzeichnungssystems aus Fig. 8 mit eingesetztem, als Implantatschraube ausgebildeten Implantat;
- Fig. 10: eine Draufsicht eines Datenträgermagazins, welches mehrere plättchenförmige Datenträger aufweist;
- Fig. 11: eine Schnittdarstellung XI-XI des Datenträgermagazins aus Fig. 10 mit mehreren als Implantatschrauben ausgebildeten Implantaten;
- Fig. 12: das Datenträgermagazin aus den Fig. 10 und 11 in perspektivischer Darstellung mit geöffneten Datenträgern;
- Fig. 13: eine zweite Ausführungsvariante eines Datenträgermagazins in perspektivischer Darstellung;
- Fig. 14: eine Seitenansicht XIV des Datenträgermagazins aus Fig. 12;
- Fig. 15: eine Seitenansicht XV des Datenträgermagazins aus Fig. 13;

Fig. 1 zeigt eine perspektivische Darstellung einer ersten Ausführungsvariante eines Kennzeichnungssystems 1, welches zur Aufnahme eines als Implantatschraube 2 ausgebildetes Implantats dient. Dieses Kennzeichnungssystem 1 besteht aus einer Aufnahmehülse 3, welche nach unten hin eine radial verjüngt ausgebildete Steckhülse 4 bildet. Im oberen Endbereich ist auf die Steckhülse 4 ein radial erweitertes Kopfteil 5 aufgesetzt, welches einstückig mit der Steckhülse 4 in Verbindung steht. Wie aus der Schnittdarstellung der Fig. 2 ersichtlich ist, weist diese Aufnahmehülse 3 eine mehrfach abgesetzte Durchgangsbohrung 6 auf, welche im Bereich des Kopfteiles 5 mit einem radial erweiterten Bohrungsabschnitt 7 versehen ist. Dieser Bohrungsabschnitt 7 dient zur Aufnahme eines Schraubenkopfes 8 der Implantatschraube 2. Wie aus Fig. 2 ersichtlich ist, stützt sich der Schraubenkopf 8 in der Durchgangsbohrung 6 an einem radial nach innen vorstehenden Stützsteg 9 axial ab, so dass die Implantatschraube 2 gegen Herausfallen aus der Aufnahmehülse 3 in Richtung des Pfeiles 10 gesichert ist.

Des Weiteren ist aus Fig. 1 und 2 ersichtlich, dass die Aufnahmehülse 3 im Umgebungsbereich des radial erweiterten Bohrungsabschnittes 7 einen vertikal über die obere Ringfläche 11 des Kopfteiles 5 nach oben überstehenden Haltesteg 12 aufweist. Auf diesen Haltesteg 12 ist ein etwa plättchenförmig ausgebildeter Datenträger 13 aufgesetzt, welcher auf der Oberseite des Haltesteges 12 beispielsweise durch eine Kleb- oder Schweißverbindung gehalten ist.

Des Weiteren ist aus den Fig. 1 und 2 ersichtlich, dass der Datenträger 13 im Bereich des Haltesteges 12 oberseitig mit einer quer verlaufenden Kerbe 14 versehen ist, so dass in diesem Bereich der Kerbe 14 ein filmscharnierartig ausgebildeter Verbindungsabschnitt 15 zwischen dem auf den Haltesteg 12 aufgesetzten und mit diesem feststehend verbundenen Halteabschnitt 16 des Datenträgers 13 und einem plättchenförmig ausgebildeten, seitlich über das Kopfteil 5 der Aufnahmehülse 3 vorstehenden Datenträgerabschnitt 17 gebildet wird. Dieser Datenträgerabschnitt 17 weist auf seiner Oberseite einer Beschriftungsfläche 18 mit einer Beschriftung 19 auf, durch welche die aufgenommene Implantatschraube 2 bezüglich ihrer Größe, Charge, ihres Herstellers und sonstiger Daten gekennzeichnet ist.

Weiter ist aus Fig. 2 ersichtlich, dass zwischen dem Datenträger 13 bzw. dessen Datenabschnitt 17 und der obern Ringfläche 11 ein Luftspalt 20 gebildet wird. Dieser Luftspalt 20 weist dementsprechend auch einen Abstand zum Bohrungsabschnitt 7 auf, so dass zum Zwecke der Sterilisation der Implantatschraube 2 auch deren Schraubenkopf 8 mit Sterilisationsmittel beaufschlagt werden kann.

Beim dargestellten Kennzeichnungssystem 1 ist der Datenabschnitt 17 des Datenträgers 13 aufgrund des geschwächt ausgebildeten Verbindungsabschnittes 15 vom Halteabschnitt 16 und damit von der Aufnahmehülse 3 manuell in einfacher Weise abreißbar. Dadurch wird gleichzeitig die Implantatschraube 2 freigegeben, so dass diese entgegen des Pfeiles 10 aus der Aufnahmehülse 3 herausnehmbar ist. Nach dem Abtrennen des Datenabschnittes 17 kann dieser aufgrund seiner äußerst "flachen", plättchenartigen Ausgestaltung in einfacher Weise in einer Patientenakte oder einem Patientendokument archiviert werden. Hierzu ist es beispielsweise möglich, diesen Datenabschnitt 17 des Datenträgers 13 in die Patientenakte oder auf das Patientendokument einzukleben bzw. aufzukleben. Das Kennzeichnungssystem 1 sowie auch die weiter noch beschriebenen Kennzeichnungssysteme sind vorzugsweise aus Kunststoff hergestellt, so dass insbesondere das Abreißen des Datenabschnittes 17 des Datenträgers 13 vom Halteabschnitt 16 in einfacher Weise manuell durchführbar ist.

Fig. 3 zeigt eine weitere Ausführungsvariante eines Kennzeichnungssystems 1/1, bei welchem für identische oder gleich wirkende Teile dieselben Bezugszeichen eingetragen sind wie bei der Ausführungsvariante des Kennzeichnungssystems 1 aus den Fig. 1 und 2. Es ist erkennbar, dass der Datenträger 13 auf seiner Oberseite ebenfalls mit einer Beschriftung 19 versehen ist. Die Aufnahmehülse 3 weist ebenfalls eine radial verjüngt ausgebildete Steckhülse 4 auf, an welcher sich oberseitig ein radial erweitertes Kopfteil 5 anschließt. Bei dieser Ausführungsvariante des Kennzeichnungssystem 1/1 ist der Datenträger 13 auf zwei axial nach oben über die obere Ringfläche 11 des Kopfteiles 5 hinaus stehende Haltestege 12 bzw. 21 aufgesetzt. Auch hier besteht zwischen dem Datenträger 13 und dieser Ringfläche 11 des Kopfteiles 5 ein Luftspalt 20, wie dies insbesondere aus der Schnittdarstellung der Fig. 4 erkennbar ist. Auch die Aufnahmehülse 3 weist eine Durchgangsbohrung 6 mit einem oberen, radial erweiterten Bohrungsabschnitt 7 auf. In diesem radial erweiterten Bohrungsabschnitt 7 wird hier ebenfalls der Schraubenkopf 8 einer Implantatschraube 2 aufgenommen. Dieser Schraubenkopf 8 stützt sich vertikal in Richtung des Pfeiles 10 nach unten an einem radial nach innen vorstehenden Stützsteg 9 der Bohrung 6 ab. Somit wird die in die Bohrung 6 eingesetzte Implantatschraube 2 durch diesen Stützsteg 9 gegen Herausfallen in Richtung des Pfeiles 10 gesichert.

Der Datenträger 13 ist auch bei dieser Ausführungsvariante im Abstand zur oberen Ringfläche 11 des Kopfteiles 5 angeordnet und deckt die Durchgangsbohrung 6 oberseitig vollständig ab. Dadurch ist die Implantatschraube 2 gegen Herausfallen in entgegengesetzter Richtung zum Pfeil 10 in der Durchgangsbohrung 6 gesichert.

Die Verbindung des Datenträgers 13 mit der Aufnahmehülse 3 besteht bei der Ausführungsvariante nach Fig. 3 aus zwei "Schweißpunkten" 22, über welche der Datenträger 13 mit dem Haltesteg 12 feststehend in Verbindung steht. Diese Schweißpunkte 22 sind derart ausgestaltet, dass diese durch Anheben des Datenträgers 13 entgegen des Pfeiles 10 aufgebrochen werden können, so dass die Durchgangsbohrung 6 bzw. deren radial erweiterter Bohrungsabschnitt 7 freigegeben wird. Nach dem Abtrennen des Datenträgers 13 durch Aufbrechen der beiden Schweißpunkte 22 wird somit die Durchgangsbohrung 6 nach oben freigegeben, so dass die Implantatschraube 2 aus der Aufnahmehülse 3 herausnehmbar ist. Der Datenträger 13 kann nach dem Abtrennen aufgrund seiner äußerst flachen, plättchenartigen Ausbildung ebenfalls in einfacher Weise zur Archivierung unverlierbar an oder auf einem Patientendokument befestigt werden. Damit ist eine eindeutige Zuordnung des entsprechenden beim Patienten eingesetzten Implantats in Form der Implantatschraube 2 stets gewährleistet. Fig. 5 zeigt hierzu die Aufnahmehülse 3 mit abgerissenem Datenträger 13. Es ist deutlich erkennbar, dass die beiden Haltestege 12 und 21 vertikal über die Ringfläche 11 des Kopfteiles 5 hinaus stehen.

Durch das Abreißen des Datenträgers 13 bzw. des Datenabschnittes 17 wird des Weiteren sichergestellt, dass dieser Datenträger 13 bzw. der Datenabschnitt 17 nicht wieder einem anderen Implantat bzw. einer anderen Implantatschraube 2 zugeordnet werden kann. D.h., dass auch hier durch das Abtrennen des Datenträgers 13 bzw. Datenabschnittes 17 sichergestellt ist, dass das Kennzeichnungssystem 1 bzw. 1/1 nicht erneut mit einem Implantat bzw. einer Implantatschraube 2 bestückt wird. Insoweit bildet das Kennzeichnungssystem 1 bzw. 1/1 in vielerlei Hinsicht eine äußerst sichere Kennzeichnung einer Implantatschraube 2, da diese einerseits durch die Abdeckung der Durchgangsbohrung 6 durch den Datenträger 13 nicht aus dem Kennzeichnungssystem 1 bzw.1/1 entnehmbar ist, ohne dass dies für den Operateur erkennbar wäre.

Insoweit ist die Kennzeichnung durch dieses Kennzeichnungssystem 1 bzw. 1/1 insbesondere einer Implantatschraube 2 stets eindeutig und kann nicht zu Verwechslungen führen. Da der Datenträger 13 aufgrund seiner plättchenförmigen Ausgestaltung unverlierbar mit dem Patientendokument verbindbar ist, ist auch nach dem Einsetzen der Implantatschraube 2 in den Körper eines Patienten eine einwandfreie und unverwechselbare Zuordnung gewährleistet. Insbesondere können hier auch keine Übertragungsfehler der Kennzeichnungsdaten 19 zum Patienten bzw. zum implantierten Implantat 2 auftreten.

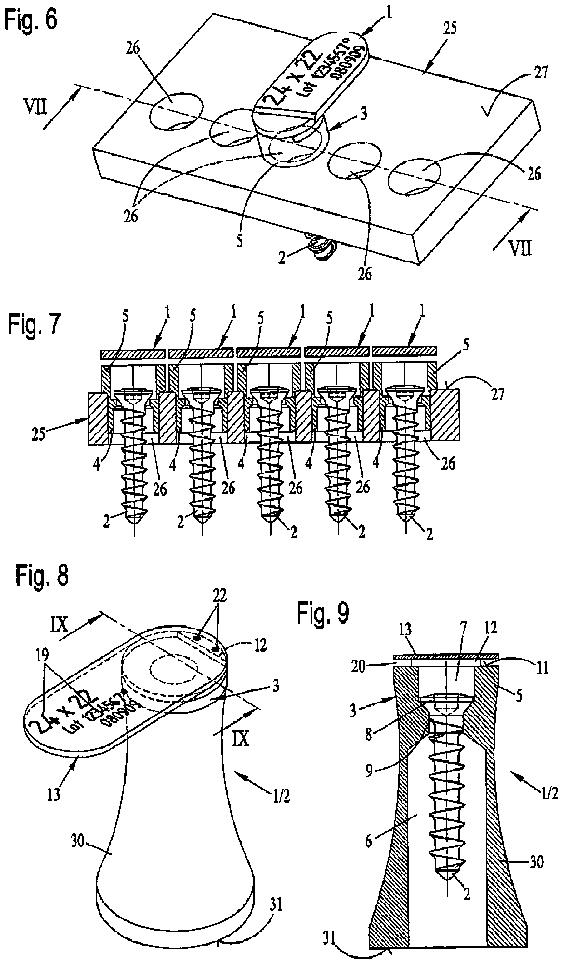

Durch die spezielle Ausgestaltung der Kennzeichnungssysteme 1 und 1/1 und insbesondere deren Aufnahmehülse 3 sind diese in einfacher Weise magazinierbar. So zeigt Fig. 6 eine perspektivische Darstellung eines plattenförmigen Aufnahmemagazins 25, welches bei der dargestellten Ausführungsvariante mit insgesamt fünf in einer Reihe angeordneten Aufnahmebohrungen 26 versehen ist. Ein solches Aufnahmemagazin 25 dient zur Bereitstellung mehrerer Implantate, insbesondere Implantatschrauben 2, während einer Operation. Wie aus Fig. 6 ersichtlich ist, ist in die mittlere Aufnahmebohrung 26 ein Kennzeichnungssystem 1 aus Fig. 1 eingesetzt. Der Durchmesser dieser Aufnahmebohrung 26 entspricht dem Durchmesser der Steckhülse 4 (Fig. 1), so dass die Aufnahmehülse 3 passend in eine der Aufnahmebohrungen 26 einsteckbar ist. Dabei stützt sich die Aufnahmehülse 3 mit ihrem radial erweiterten Kopfteil 5 auf der Oberfläche 27 des Aufnahmemagazins 25 im Umgebungsbereich der zugehörigen Aufnahmebohrung 26 axial ab. Es ist leicht vorstellbar, dass mehrere solcher Kennzeichnungssysteme 1 in das Aufnahmemagazin 25 einsetzbar sind.

Hierzu zeigt Fig. 7 eine entsprechende Schnittdarstellung VII-VII aus Fig. 6. Es ist erkennbar, dass insgesamt fünf Kennzeichnungssysteme 1 in die jeweils zugehörige Aufnahmebohrung 26 des Aufnahmemagazins 25 mit ihrer jeweiligen Steckhülse 4 eingesetzt sind. Mit ihren radial erweiterten Kopfteilen 5 stützt sich jedes der Kennzeichnungssysteme 1 auf der Oberfläche 27 des Aufnahmemagazins 25 ab. Bei der dargestellten Ausführungsvariante des Aufnahmemagazins 25 durchragen die in den Kennzeichnungssystemen 1 aufgenommenen Implantatschrauben 2 das Aufnahmemagazin 25 vertikal nach unten. Insoweit kann das plattenförmige Aufnahmemagazin 25 in eine weitere entsprechend ausgebildete Aufnahmeeinrichtung einsetzbar sein, so dass die Kennzeichnungssysteme 1 mit ihren aufgenommenen Implantatschrauben 2 nicht aus den Aufnahmebohrungen 26 des Aufnahmemagazins 25 herausgedrückt werden. Auch ist eine senkrechte Ausrichtung des Aufnahmemagazins 25 denkbar.

Fig. 8 zeigt eine perspektivische Darstellung einer weiteren Ausführungsvariante eines Kennzeichnungssystems 1/2. Dieses Kennzeichnungssystem 1/2 weist ebenfalls einen Datenträger 13 auf, auf dessen Oberseite ebenfalls eine Beschriftung 19 angebracht ist. Dieser Datenträger 13 ist, in ähnlicher Weise wie der Datenträger 13 nach der Ausführungsvariante des Kennzeichnungssystems 1/1 aus Fig. 3, über zwei Schweißpunkte 22 mit der Aufnahmehülse 3 abreißbar verbunden. Auch der Datenträger 13 der Ausführungsvariante des Kennzeichnungssystems 1/2 weist zur oberen Ringfläche 11 des Kopfteiles 5 der Aufnahmehülse 3 einen Abstand auf, so dass zwischen dem Datenträger 13 und der Ringfläche 11 ebenfalls ein Luftspalt 20 gebildet wird, dessen Größe dem vertikalen Überstand nach oben des Haltesteges 12 entspricht. Weiter ist insbesondere aus der Schnittdarstellung IX-IX der Fig. 9 erkennbar, dass auch die Aufnahmehülse 3 eine Durchgangsbohrung 6 aufweist, welche ebenfalls abgesetzt ausgebildet ist. Die Implantatschraube 2 ist in diese Durchgangsbohrung 6 eingesetzt, wobei deren Schraubenkopf 8 in einem radial erweiterten Abschnitt 7 der Durchgangsbohrung 6 aufgenommen wird. Diese Ausführungsvariante des Kennzeichnungssystems 1/2 weist in vertikal nach unten gerichteter Verlängerung zur Aufnahmehülse 3 einen Standfuß 30 auf, dessen Länge derart ausgebildet ist, dass die eingesetzte Implantatschraube 2 nicht aus der Durchgangsbohrung 6 vertikal nach unten herausragen kann. Somit bildet dieser Standfuß 30 unterseitig eine ringförmige Standfläche 31, mit welcher das Kennzeichnungssystem 1/2 auf einem ebenen Untergrund aufstellbar ist.

Des Weiteren ist aus Fig. 9 erkennbar, dass die Durchgangsbohrung 6 unterhalb des radial erweiterten Bohrungsabschnittes 7 einen radial nach innen vorstehenden Stützsteg 9 bildet, an welchem sich der Schraubenkopf 8 der Implantatschraube 2 vertikal abstützt. Das Kennzeichnungssystem 1/2 ist insbesondere vorgesehen, um Implantatschrauben 2 als "Einzelstücke" dem Operateur zur Verfügung zu stellen.

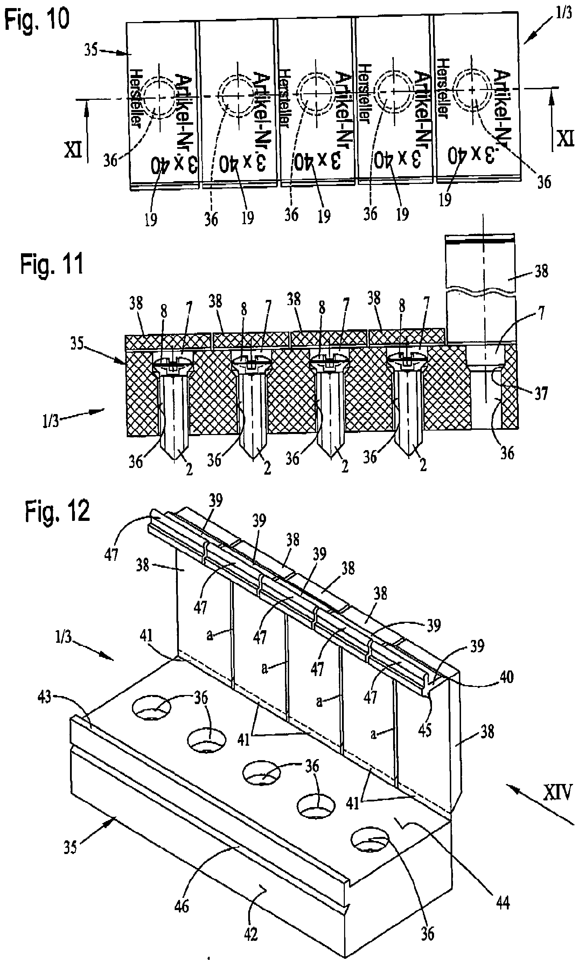

Fig. 10 zeigt eine Draufsicht einer weiteren Variante eines Kennzeichnungssystems 1/3. Dieses Kennzeichnungssystem 1/3 ist als eine Art Datenmagazin 35 ausgebildet, welches mehrere Durchgangsbohrungen 36 aufweist. In diese Durchgangsbohrungen 36 ist jeweils eine Implantatschraube 2 einsetzbar, wie dies aus der Schnittdarstellung XI-XI der Fig. 11 ersichtlich ist. Weiter ist aus Fig. 11 ersichtlich, dass jede der Durchgangsbohrungen 36 ebenfalls einen oberen, radial erweiterten Bohrungsabschnitt 7 aufweist, in welchem jeweils der entsprechende Schraubenkopf 8 der zugehörigen Implantatschraube 2 passend aufgenommen wird. Aufgrund der radial erweiterten Ausbildung des Bohrungsabschnittes 7 bildet die Durchgangsbohrung 36 einen radial nach innen gerichteten Anschlagbund 37, an welchem sich der jeweilige Schraubenkopf 8 im angesetzten Zustand vertikal nach unten abstützt.

Weiter ist aus den Fig. 10 und 11 erkennbar, dass die Durchgangsbohrungen 36 jeweils mittels eines separaten Datenträgers 38 abgedeckt sind. Diese Datenträger 38 sind jeweils mit einer entsprechenden Beschriftung 19 versehen, wie dies aus Fig. 10 ersichtlich ist. Im geschlossenen Zustand werden die Implantatschrauben 2 in der jeweiligen Durchgangsbohrung 36 durch den jeweils zugehörigen Datenträger 38 unverlierbar gesichert. Dabei zeigt Fig. 11 einen teilweise geöffneten Zustand eines der Datenträger 38, wobei die diesbezüglich zugeordnete Implantatschraube bereits herausgenommen ist. Nachdem die Implantatschraube 2 herausgenommen ist, kann der Datenträger 38 in einfacher Weise vom Datenmagazin 35 abgenommen werden.

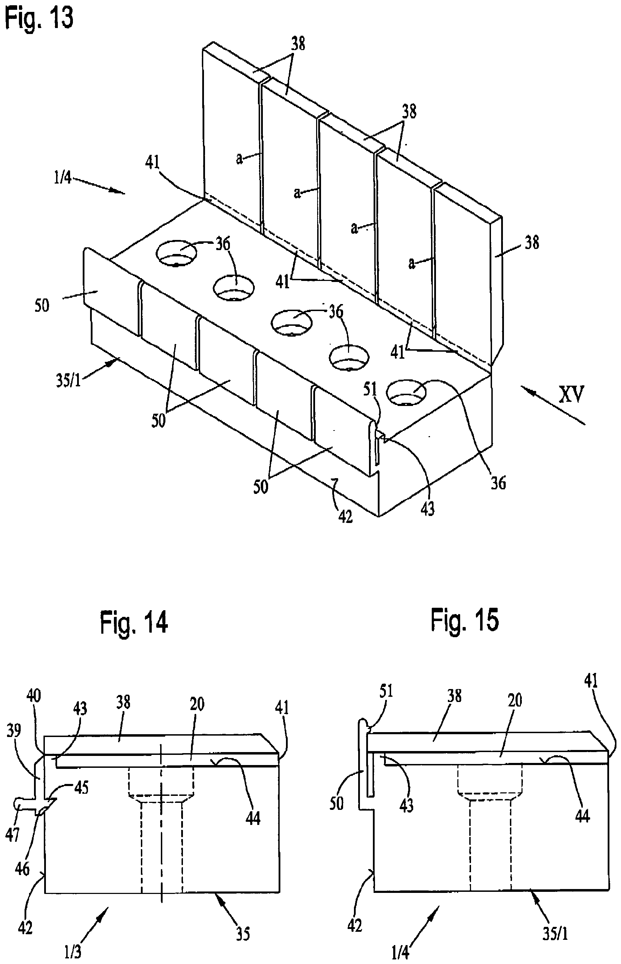

Hierzu zeigt Fig. 12 das Datenmagazin 35 in perspektivischer Darstellung. Alle Datenträger 38 sind in der Darstellung der Fig. 12 in ihrem geöffneten Zustand dargestellt. Weiter ist aus Fig. 12 ersichtlich, dass jeder Datenträger 38 eine Rastlasche 39 aufweist, welche über eine Art Filmscharnier 40 abreißbar am jeweiligen Datenträger 38 angeordnet ist. Jeder der Datenträger 38 ist des Weiteren über ein weiteres, den Rastlaschen 39 gegenüberliegendes Filmscharnier 41 mit dem Datenmagazin 35 verbunden. Des Weiteren bildet das Datenmagazin 35, welches blockartig ausgebildet ist, im Bereich seiner vorderen Stirnseite 42 einen vertikal nach oben vorstehenden Haltesteg 43, auf welchem die Datenträger 38 in ihrem geschlossenen Zustand aufliegen. Damit wird zwischen dem jeweiligen Datenträger 38 und der Oberfläche 44 des Datenmagazins 35 ebenfalls ein Luftspalt 20 gebildet, so dass durch diesen Luftspalt 20 zur Sterilisation der aufgenommenen Implantatschraube 2 ausreichend Sterilisationsmittel, insbesondere zum Schraubenkopf 8 der Implantatschraube 2, gelangen kann (Fig. 14). Zu Verbesserung dieser Zufuhr weisen die Datenträger 38 untereinander eine seitlichen Abstand a voneinander auf.

Des Weiteren ist aus Fig. 14 ersichtlich, dass die Rastlaschen 39 eine Rastnase 45 bilden, mit welcher diese rastend mit einer entsprechenden Rastnut 46 des Datenmagazins 35 in Eingriff bringbar sind. Diese Rastnut 46 erstreckt sich dabei, wie dies aus Fig. 12 ersichtlich ist, über die komplette Länge der vorderen Stirnseite 42 des Datenmagazins 35. Weiter ist aus den Fig. 12 und 14 noch ersichtlich, dass in den Rastlaschen 39 etwa im Bereich ihrer Rastnasen 45 jeweils mit einer Betätigungsleiste 47 versehen sind, welche zur leichteren Handhabung beim Entriegeln dieser Rastlaschen 39 dient.

Nach dem Öffnen des jeweiligen Datenträgers 38 kann die jeweils aufgenommene Implantatsschraube 2 aus der zugehörigen Durchgangsbohrung 36 herausgenommen werden. Anschließend wird der Datenträger 38 durch Abreißen des Filmscharniers 41 vom Datenträgermagazin 35 abgetrennt. Um diesen Datenträger 38 nun in einfacher Weise auf einem Patientendokument anbringen zu können, kann die Rastlasche 39 über das Filmscharnier 40 ebenfalls manuell vom Datenträger 38 abgetrennt werden, so dass dieser Datenträger 38 anschließend ein ebenes plättchenartiges Element bildet, welches beispielsweise auf einem Patientendokument aufgeklebt werden kann.

Fig. 13 zeigt eine weitere Ausführungsvariante eines Kennzeichnungssystems 1/4 in Form eines Datenmagazins 35/1, wobei in Fig. 13 für die gleichen Teile die gleichen Bezugszeichen eingetragen sind wie bei der Ausführungsvariante des Datenmagazins 35 aus Fig. 12, so dass die Beschreibung zur Ausführungsvariante der Fig. 12 insoweit auch auf Fig. 13 zu lesen ist.

Dieses Datenmagazin 35/1 weist ebenfalls mehrere Datenträger 38 auf, welche in Fig. 13 in ihrem jeweils geöffneten Zustand dargestellt sind und ebenfalls ein seitlichen Abstand a voneinander aufweisen. Die Datenträger 38 sind ebenfalls jeweils über ein Filmscharnier 41 mit dem Datenmagazin 35/1 abtrennbar verbunden. Den Filmscharnieren 41 gegenüberliegend weist auch das Datenmagazin 35/1 im Bereich seiner vorderen Stirnseite 42 einen vertikal nach oben gerichteten Haltesteg 43 auf, auf welchem die Datenträger 38 in geschlossenem Zustand aufliegen (Fig. 15). Weiter ist ebenfalls aus Fig. 15 ersichtlich, dass aufgrund der vertikalen Höhe der Filmscharniere 41 sowie des Haltesteges 43 die Datenträger 38 einen Abstand zur Oberfläche 44 des Datenmagazins 35/1 aufweisen, so dass zwischen dem Datenträger 38 und dieser Oberfläche 44 ebenfalls ein Luftspalt 20 vorhanden ist.

Die Konstruktion des Datenmagazins 35/1 unterscheidet sich von der Konstruktion des Datenmagazins 35 durch die rastende Arretierung ihrer Datenträger 38 in geschlossenem Zustand. Es ist aus Fig. 14 erkennbar, dass jedem Datenträger 38 an der vorderen Stirnseite 42 jeweils eine Rastlasche 50 zugeordnet ist. Diese Rastlasche 50 ist federelastisch mit dem Datenmagazin 35/1 verbunden. Zum geschlossenen Datenträger 38 hin bildet jede der Rastlaschen 50 eine zum jeweiligen Datenträger 38 hin vorstehende Rastnase 51, mit welcher die Rastlasche 50 im geschlossenen Zustand des jeweils zugehörigen Datenträgers 38 formschlüssig, rastend mit diesem Datenträger 38 in Eingriff steht. Diese Ausführungsvariante hat den Vorteil, dass die Rastlasche 50 mit ihrer Rastnase 51 nach dem Abreißen des jeweiligen Datenträgers 38 über dessen jeweiliges Filmscharnier 41 nicht noch zusätzlich vom Datenträger 38 entfernt werden muss. Dadurch kann der Datenträger 38 nach der Ausführungsvariante des Datenmagazins 35/1 unmittelbar nach dem Abtrennen vom Datenmagazins 35/1 auf oder an einem Patientendokument angebracht werden. Irgendwelche störend abstehenden Teile sind dementsprechend nach dem Abtrennen hier nicht mehr vorhanden.

Die Handhabung der Datenmagazine 35 und 35/1 aus den Fig. 12 und 13 wird nachfolgend nochmals näher erläutert:
Zum Entnehmen einer Implantatschraube 2 aus einem Magazin 35 bzw. 35/1 wird zuerst die jeweilige Rastlasche 39 bzw. 50 des entsprechenden Datenträgers 38 gelöst. Anschließend wird der Datenträger 38 aus seiner die Implantatschraube 2 überdeckenden Lage durch Verschwenken nach oben geöffnet. Danach kann die betreffende Implantatschraube 2 aus der zugehörigen Durchgangbohrung 36 des Magazins 35 bzw. 35/1 entnommen werden. Anschließend wird der dieser Implantatschraube 2 zugeordnete Datenträger 38 durch Abreißen des Filmscharniers 41 vom Datenmagazin 35 bzw. 35/1 abgetrennt und umgehend auf ein Datenblatt der Patientenakte des gerade behandelten Patienten geheftet oder geklebt. Wie bereits erwähnt, kann dies z. B. durch einen sich bereits auf dem Datenblatt des Patienten befindenden Klebstreifen oder mittels einer Klammer erfolgen. In jedem Falle muss eine dauerhaft haltbare Verbindung zwischen dem Datenträger 38 und dem Datenblatt gewährleistet sein.

Bei allen Ausführungsformen steht für die Kennzeichnung der Implantate eine ausreichend große Beschriftungsflächen für die zu speichernden Daten zur Verfügung. Damit können die erforderlichen, das jeweilige Implantat betreffenden Daten in einer gut leserlichen Schriftgröße auf den jeweils zugehörigen Datenträger gebracht werden. Eine solche Beschriftung kann, wie dies beispielhaft in den Zeichnungen dargestellt ist, in "Textform" auf dem entsprechenden Datenträger angebracht sein. Es sind jedoch auch andere Datenkennzeichnungsarten denkbar. So können die Kenndaten für das Implantat auch in Form einer Datamatrix oder im RFID-Format auf dem Datenträger angebracht sein. Auch ist ein Datenchip, beispielsweise in Form eines auslesbaren Transponders, denkbar, welcher als separates Bauteil im Datenträger integriert oder auf den Datenträger aufgeklebt ist.

Damit ist es nicht erforderlich, die zu archivierenden Daten zum jeweiligen Implantat durch Abschrift in die Patientenakte aufzunehmen. So wird eine häufige Fehlerquelle bei der Archivierung und Zuordnung dieser Daten zur Patientenakte ausgeschlossen. Es kann folglich auch keine Übertragungsfehler geben. Dabei ist es von untergeordneter Bedeutung, welche konkrete äußere Form die Datenträger im Einzelnen haben und in welcher Weise die Daten auf diesen aufgebracht sind. Diese können beispielsweise unlöschbar aufgedruckt oder aufgeprägt sein.

## Patentansprüche

1. Kennzeichnungssystem (1, 1/1, 1/2, 1/3, 1/4) für medizinische Implantate (2), bei dem auf einer Beschriftungsfläche (18) produktspezifische Daten (19), wie z.B. Größe, Hersteller, Charge oder sonstige Kennzeichnungsdaten unlöschbar aufgebracht sind, anhand derer die Herkunft oder die Eigenschaften oder die Herkunft und die Eigenschaften des betreffenden Implantats (2, 75) rückverfolgbar sind, wobei sich die Beschriftungsfläche (18) auf einem Datenträger (13, 38) befindet, der die Form eines Plättchens, Schildchens oder Streifens aufweist, wobei der abgetrennte Datenträger (13, 38) an oder auf einem Patientendokument mittels einer Kleb- oder einer Klammerverbindung unverlierbar befestigbar ist; **dadurch gekennzeichnet,**
**dass** der Datenträger (13, 38) manuell abtrennbar mit einem das Implantat (2) aufnehmenden Lagerelement (3, 35, 35/1) verbunden ist und das Implantat (2) im Lagerelement (3, 35, 35/1) durch den Datenträger (13, 18) unverlierbar gesichert ist.

2. Kennzeichnungssystem nach Anspruch 1, **dadurch gekennzeichnet, dass** das Lagerelement hülsenförmig ausgebildet ist und eine Aufnahmehülse (3) mit einer abgesetzten Aufnahmebohrung (6) für ein als Implantatschraube (2) ausgebildetes Implantat aufweist und,
dass die das Implantat (2) aufnehmende Aufnahmebohrung (6) durch den Datenträger (13) abgedeckt ist und,
dass der Datenträger (13) über einen abreißbaren Verbindungsabschnitt (15) mit der Aufnahmehülse (3) in Verbindung steht.

3. Kennzeichnungssystem nach Anspruch 2, **dadurch gekennzeichnet, dass** die Aufnahmehülse (3) eine Steckhülse (4) mit einem radial erweiterten Kopfteil (5) bildet und, dass der Datenträger (13) auf dem Kopfteil (5), die Aufnahmebohrung (6) abdeckend angeordnet ist und, dass die Aufnahmehülse (3) mit ihrer Steckhülse (4) in eine von mehreren Aufnahmebohrungen (26) eines Aufnahmemagazins (25) einsteckbar ist.

4. Kennzeichnungssystem nach Anspruch 2 oder 3, **dadurch gekennzeichnet, dass** der Verbindungsabschnitt (15) zwischen dem Datenträger (13) und der Aufnahmehülse (3) durch eine kerbenartige Querschnittsverjüngung (14) gebildet und als eine Art Filmscharnier ausgebildet ist.

5. Kennzeichnungssystem nach Anspruch 2, 3 oder 4, **dadurch gekennzeichnet, dass** die Aufnahmehülse (3) einen axial verlängerten Standfuß (30) mit einer unterseitigen, im Wesentlichen ebenen, etwa ringförmig ausgebildeten Standfläche (31) aufweist.

6. Kennzeichnungssystem nach Anspruch 1, **dadurch gekennzeichnet, dass** das Lagerelement zur Aufnahme mehrerer Implantate in Form von Implantatschrauben (2) als Datenträgermagazin (35, 35/1) ausgebildet ist, das mehrere die Implantate (2) aufnehmende Durchgangsbohrungen (36) aufweist, welche oberseitig durch einzelne, separat abtrennbare, den in den Durchgangsbohrungen (36) befindlichen Implantatschrauben (2) jeweils zugeordnete Datenträger (38) abgedeckt sind.

7. Kennzeichnungssystem nach Anspruch 6, **dadurch gekennzeichnet, dass** der der jeweiligen Durchgangsbohrung (36) des aus Kunststoff bestehenden Datenträgermagazins (35, 35/1) zu deren Abdeckung zugeordnete Datenträger (38) mittels eines Filmscharniers (41) an einer Längskante eines das Datenträgermagazin (35, 35/1) bildenden Blocks aufklappbar und abreißbar angelenkt ist.

8. Kennzeichnungssystem nach Anspruch 6 oder 7, **dadurch gekennzeichnet, dass** die Datenträger (38) des Datenträgermagazins (35) mittels einer abreißbaren Rastlasche (39) in ihrer Schließlage am Datenträgermagazin (35) verriegelbar sind.

9. Kennzeichnungssystem nach Anspruch 6 oder 7, **dadurch gekennzeichnet, dass** die als schwenkbarer Deckel ausgebildeten Datenträger (38) des Datenträgermagazins (35/1) jeweils mittels einer an der Stirnfläche (42) des Datenträgermagazins (35/1) angeordneten Ratlasche (50) in ihrer Schließlage fixierbar sind, wobei die Rastlasche (50) den jeweils zugeordneten Datenträger (38) mit einer Rastnase (51) übergreift.

10. Kennzeichnungssystem nach der Anspruch 1, **dadurch gekennzeichnet, dass** der Datenträger (13, 38) aus Kunststoff oder einem metallischen Werkstoff besteht.

## Claims

1. Identification system (1, 1/1, 1/2, 1/3, 1/4) for medical implants (2), in which product-specific data (19), e.g. size, manufacturer, batch or other identification data, are applied indelibly to an inscription surface (18), on the basis of which data the origin or the properties or the origin and the properties of the relevant implant (2, 75) are traceable, wherein the inscription surface (18) is located on a data carrier (13, 38), which has the form of a plaque, tag or strip, wherein the separated data carrier (13, 38) can be fastened securely at or on a patient document by means of an adhesive connection or a stapled connection, **characterized in that** the data carrier (13, 38) is connected in a manually separable manner to a bearing element (3, 35, 35/1) receiving the implant (2), and the implant (2) is secured captively in the bearing element (3, 35, 35/1) by the data carrier (13, 18).

2. Identification system according to Claim 1, **characterized in that** the bearing element is sleeve-shaped and has a receiving sleeve (3) with a stepped receiving bore (6) for an implant configured as an implant screw (2), and **in that** the receiving bore (6) receiving the implant (2) is covered by the data carrier (13), and **in that** the data carrier (13) is connected to the receiving sleeve (3) via a tearable connection portion (15).

3. Identification system according to Claim 2, **characterized in that** the receiving sleeve (3) forms a plug-in sleeve (4) with a radially widened head part (5), and **in that** the data carrier (13) is arranged on the head part (5), covering the receiving bore (6), and **in that** the receiving sleeve (3) is insertable with its plug-in sleeve (4) into one of several receiving bores (26) of a receiving magazine (25).

4. Identification system according to Claim 2 or 3, **characterized in that** the connection portion (15) between the data carrier (13) and the receiving sleeve (3) is formed by a notch-like cross-sectional narrowing (14) and is configured as a kind of film hinge.

5. Identification system according to Claim 2, 3 or 4, **characterized in that** the receiving sleeve (3) has an axially prolonged standing base (30) with, on the underside thereof, a substantially plane and approximately annular standing surface (31).

6. Identification system according to Claim 1, **characterized in that** the bearing element is configured as a data carrier magazine (35, 35/1) for receiving a plurality of implants in the form of implant screws (2), said data carrier magazine (35, 35/1) having a plurality of through-bores (36) which receive the implants (2) and which are covered at the top by individual, separately detachable data carriers (38) which are respectively assigned to the implant screws (2) located in the through-bores (36).

7. Identification system according to Claim 6, **characterized in that** the data carrier (38), assigned to and covering the respective through-bore (36) of the data carrier magazine (35, 35/1) made of plastic, is articulated by means of a film hinge (41), at a longitudinal edge of a block forming the data carrier magazine (35, 35/1), in such a way that it can be folded open and torn off.

8. Identification system according to Claim 6 or 7, **characterized in that** the data carriers (38) of the data carrier magazine (35) can be locked in their closure position on the data carrier magazine (35) by means of a tear-off latching tab (39) .

9. Identification system according to Claim 6 or 7, **characterized in that** the data carriers (38) of the data carrier magazine (35/1) are configured as a pivotable lid and can each be fixed in their closure position by means of latching tab (50) arranged on the front face (42) of the data carrier magazine (35/1), wherein a latching lug (51) of the latching tab (50) engages over the respectively associated data carrier (38).

10. Identification system according to Claim 1, **characterized in that** the data carrier (13, 38) is made of plastic or of a metallic material.

## Revendications

1. Système d'identification (1, 1/1, 1/2, 1/3, 1/4) pour implants médicaux (2), dans lequel des données spécifiques du produit (19), telles que la taille, le fabricant, le lot ou d'autres données d'identification, sont appliquées de manière indélébile sur une surface de marquage (18), par le biais desquelles la provenance ou les propriétés, ou la provenance et les propriétés de l'implant (2, 75) concerné peuvent être retrouvées, dans lequel la surface de marquage (18) est située sur un support de données (13, 38) qui présente la forme d'une plaque, d'une étiquette ou d'une bande, dans lequel le support de données séparé (13, 38) peut être fixé de manière imperdable à ou sur un document de patient au moyen d'une liaison par adhésif ou par agrafage ;
**caractérisé en ce que** le support de données (13, 38) est relié manuellement de manière séparable à un élément de support (3, 35, 35/1) recevant l'implant (2) et **en ce que** l'implant (2) est fixé de manière imperdable dans l'élément de support (3, 35, 35/1) par le support de données (13, 18).

2. Système d'identification selon la revendication 1, **caractérisé en ce que** l'élément de support est réalisé en forme de manchon et comporte un manchon de réception (3) muni d'un alésage de réception (6) en gradins destiné à un implant réalisé sous la forme d'une vis d'implant (2) et
**en ce que** l'alésage de réception (6) recevant l'implant (2) est recouvert par le support de données (13) et
**en ce que** le support de données (13) est relié au manchon de réception (3) par l'intermédiaire d'une section de raccordement (15) arrachable.

3. Système d'identification selon la revendication 2, **caractérisé en ce que** le manchon de réception (3) forme un manchon enfichable (4) muni d'une partie de tête (5) élargie radialement, et **en ce que** le support de données (13) est disposé sur la partie de tête (5) de manière à recouvrir l'alésage de réception (6), et **en ce que** le manchon de réception (3) peut être enfiché par son manchon enfichable (4) dans l'un de plusieurs alésages de réception (26) d'un magasin de réception (25).

4. Système d'identification selon la revendication 2 ou 3, **caractérisé en ce que** la section de raccordement (15) -est formée entre le support de données (13) et le manchon de réception (3) par un rétrécissement de section transversale (14) en forme d'encoche et est réalisée sous la forme d'un type de charnière à film.

5. Système d'identification selon la revendication 2, 3 ou 4, **caractérisé en ce que** le manchon de réception (3) présente sur sa face inférieure une embase allongée axialement (30) qui présente une surface d'appui sensiblement plane et approximativement annulaire (31).

6. Système d'identification selon la revendication 1, **caractérisé en ce que** l'élément de support destiné à recevoir plusieurs implants sous la forme de vis d'implant (2) est réalisé sous la forme d'un magasin de supports de données (35, 35/1) qui comporte plusieurs alésages traversants (36) recevant les implants (2), qui sont recouverts, sur leur face supérieure, par des supports de données (38) séparables individuellement et respectivement associés aux vis d'implants (2) situées dans les alésages traversants (36).

7. Système d'identification selon la revendication 6, **caractérisé en ce que** le support de données (38) associé à l'alésage traversant (36) respectif du magasin de supports de données (35, 35/1) constitué de matière plastique pour recouvrir ce dernier est articulé de manière à pouvoir être rabattu et arraché au moyen d'une charnière à film (41) sur un bord longitudinal d'un bloc formant le magasin de supports de données (35, 35/1).

8. Système d'identification selon la revendication 6 ou 7, **caractérisé en ce que** les supports de données (38) du magasin de supports de données (35) peuvent être verrouillés dans leur position fermée sur le magasin de supports de données (35) au moyen d'une patte de verrouillage arrachable (39).

9. Système d'identification selon la revendication 6 ou 7, **caractérisé en ce que** les supports de données (38) du magasin de supports de données (35/1), qui sont réalisés sous la forme de couvercles pivotants, peuvent être fixés dans leur position fermée au moyen d'une patte de verrouillage (50) disposée sur la face avant (42) du magasin de supports de données (35/1), dans lequel la patte de verrouillage (50) s'engage par une languette d'encliquetage (51) sur le support de données (38) respectivement associé.

10. Système d'identification selon la revendication 1, **caractérisé en ce que** le support de données (13, 38) est constitué de matière plastique ou d'un matériau métallique.
